(19) Europäisches Patentamt
European Patent Office
Office européen des brevets

(11) Publication number: 0 184 133
A2

# (12) EUROPEAN PATENT APPLICATION

(21) Application number: 85115058.1

(22) Date of filing: 27.11.85

(51) Int. Cl.[4]: C 08 G 59/68

(30) Priority: 29.11.84 IT 2379284

(43) Date of publication of application: 11.06.86 Bulletin 86/24

(84) Designated Contracting States: BE DE FR GB NL

(71) Applicant: RESEM S.p.A.
13, Via Sempione
Castellanza Varese(IT)

(72) Inventor: Federici, Franco, Dr. Chem.
49, Viale Stelvio
Busto Arsizio (Varese)(IT)

(72) Inventor: Galbiati, Carlo, Dr. Chem.
3, via A Costa
Novara(IT)

(72) Inventor: Venchiarutti, Diego
17, via Nazario Sauro
Bovisio Masciago (Milano)(IT)

(74) Representative: Weinhold, Peter, Dr. et al,
Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8
D-8000 München 40(DE)

## (54) Polyamidoamines and use thereof as hardening catalysts for epoxy resins.

(57) Polyamidoamines of formula:

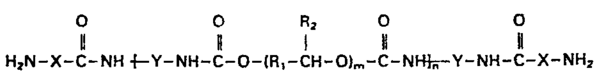

(1)

and use thereof as hardening catalysts for epoxy resins.

EP 0 184 133 A2

Croydon Printing Company Ltd.

This invention relates to polyamidoamines, to formulations of epoxy resins containing them and to the use of same in the hardening of epoxy resins.

Epoxy resins are well known and are widely employed in many industrial fields.

Generally, these resins may be defined as intermediate resinous materials which are characterized by the presence of at least two epoxy groups.

They are broadly utilized in structural applications and in protective coatings thanks to their hardness, adhesion to surfaces, chemical resistance and electrical properties. This combination of properties can be hardly found in other individual plastic materials.

Generally, the epoxy resins require, for being hardened, the addition of a catalyst to provide a thermosetting material.

Many hardeners for epoxy resins are known. Among these, a few are used for the hardening at room temperature, while others for the hardening at high temperatures.

Generally, the hardening can be accomplished either according to an addition process or by catalytic polymerization, and both the epoxy groups and the hydroxyl groups of the resin can take part therein.

Generally, the known hardeners for epoxy resins can be classified in three classes:

(1) the acid type, such as, e.g., the anhydrides;

(2) the condensation products of aldehydes, such as, e.g., the phenol-formaldehyde resins, the ureaformaldehyde resins and the melamine-formaldehyde resins;

(3) the aminic type, such as, e.g., the aliphatic and aromatic amines, the polyamides, the tertiary amines and amine adducts.

THE PRESENT INVENTION

We have now found that excellent hardeners of the aminic type for epoxy resins are the polyamidoamines having formula:

$$H_2N-X-\overset{O}{\overset{\|}{C}}-NH-\left[Y-NH-\overset{O}{\overset{\|}{C}}-O-(R_1-\overset{R_2}{\overset{|}{C}H}-O)_m-\overset{O}{\overset{\|}{C}}-NH-\right]_n Y-NH-\overset{O}{\overset{\|}{C}}-X-NH_2 \qquad (1)$$

in which: $R_1$ is an alkylene radical containing from 1 to 6 carbon atoms; $R_2$ is hydrogen or an alkyl radical containing from 1 to 3 carbon atoms; X is the radical $-\!\!\left[(CH_2)_a-NH\right]_b$ or the radical

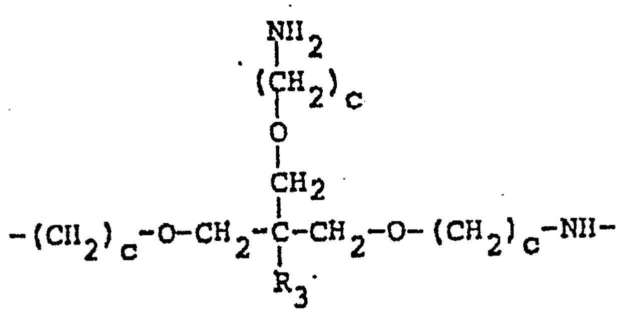

the imino group of which is bound to the carbonyl group -COin formula (1) in any case, Y is one or more of the following divalent radicals: A simple or substituted, linear or ramified aliphatic radical containing from 1 to 10 carbon atoms; a simple or substituted arylen radical containing from 6 to 20 carbon atoms; a simple or substituted cycloaliphatic radical containing from 3 to 20 carbon atoms, or an aliphatic-aromatic radical such as an alkylene-aromatic radical; m is an integer from 1 to 100,

preferably from 5 to 30; n is an integer from 0 to 10, preferably from 1 to 5; a is an integer from 2 to 6; b is an integer from 2 to 6; c is an integer from 1 to 4 and $R_3$ is an alkyl radical containing from 1 to 18 carbon atoms.

A formulation, which is also an object of the present invention, includes 100 parts by weight of epoxy resin and from 50 to 250, preferably from 80 to 180 parts by weight of a polyamidoamine having formula (1) as defined hereinabove. After hardening, such formulation exhibits excellent flexibility, high heat distortion temperatures and an excellent stability to solvents.

The polyamidoamine having formula (1) is suitably prepared by a two-step process.

The -NCO terminated polyurethane resin is prepared in the first step and, in the second step, it is reacted with the polyamine according to the scheme:

1st step:

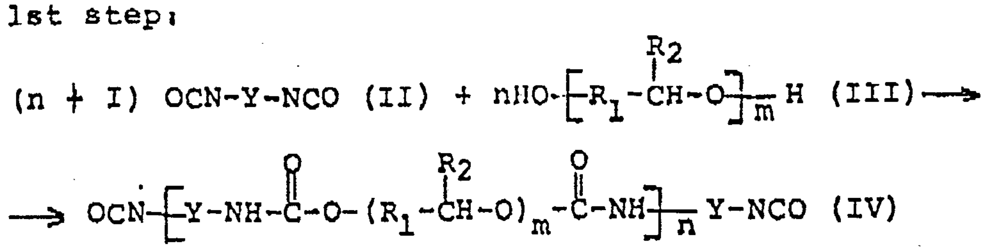

2nd step:

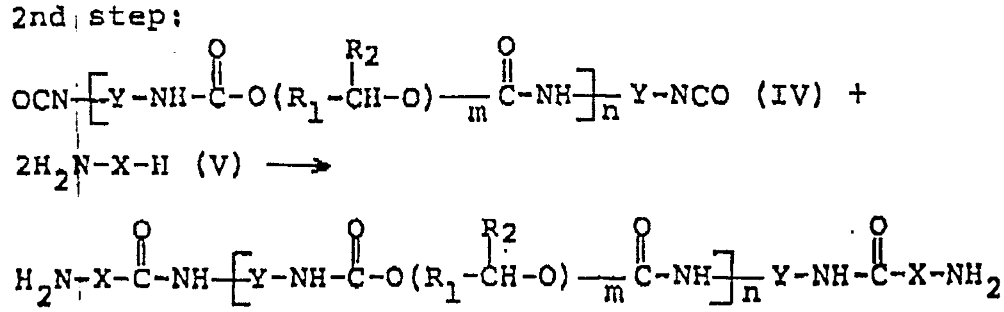

(1)

wherein X, Y, $R_1$, $R_2$, m and n have the values indicated hereinbefore.

The first process step is preferably carried out by percolating, in 1-2 hours, polyglycol (III) into isocyanate (II) at a temperature ranging from 30 to 100°C and maintaining the reaction mixture at such temperature during 3-4 hours.

The resulting polyurethane resin (IV) is then dissolved at room temperature in an organic solvent thereof, preferably an aliphatic alcohol such as methyl or ethyl alcohol, in order to obtain a solution at a concentration which ranges from 20 to 60% by weight.

In the second step, the so obtained solution containing the polyurethane resin (IV), is percolated, in 10-100 minutes and at a temperature ranging from 20 to 40°C, into a solution of polyamine (V) in an aliphatic alcohol.

The polyamidoamine of formula (1) is separated from the reaction mass by distillation of the solvent.

An important use of the polyamidoamines (1) of the present invention is that as hardener for polyepoxides or epoxy resins.

Compounds containing at least two epoxy groups, preferably terminal epoxy groups, can be hardened by using the polyamidoamines (1) of the present invention. Such polyepoxides may be saturated or unsaturated, aliphatic, cycloaliphatic, aromatic, heterocyclic and may be substituted, for example, by hydroxy groups or ether radical groups. The polyepoxides may be monomeric or polymeric and the preparation thereof is well known in the art.

Hardening of polyepoxides with the polyamidoamine (1) of the present invention can be obtained simply by mixing the two components. While the reaction between the two components can take place slowly at room temperature, better results are obtainable if the mixture is heated to 50-180°C during 1-12 hours. A subsequent post-hardening during 1-8 hours at 140-225°C may be accomplished.

Generally, it is preferable that the polyepoxide is in a stirrable form when the hardener is added, in order to facilitate a uniform mixing. If the polyepoxide is very viscous or solid at room temperature or at the forming temperature, it may be heated in order to reduce its viscosity, or a liquid volatile solvent, subsequently removable by evaporation either before or during hardening, may e added to the polyepoxide.

Examples of liquid volatile solvents are the ketones, such as acetone and methyl-ethylketone; the esters such as ethyl acetate and butyl acetate; the alcohol-ethers such as methyl, ethyl or butyl ethers of ethylene glycol; the chlorinated hydrocarbons such as chloroform, and the like.

The polyamidoamines (1) forming the object of the present invention, besides being utilized as hardeners for epoxy resins, can be used for many other purposes, for example, they are utilizable for forming compositions of diisocyanates, or for forming polyamides, etc.

The present invention is further explained by the following examples, which are given for a merely illustrative purpose and are not intended to be limiting.

In the examples, all parts, percentages and ratios are by weight, unless otherwise specified.

EXAMPLE 1

348.0 g (2 moles) of toluene diisocyanate (TDI) were charged into a 4-neck flask having a capacity of 1000 ml, equipped with stirrer, reflux cooler, dropping funnel and thermometer.

It was heated to 80°C and 400.0 g (1 mole) of polyethylene glycol having a molecular weight of 400 were added in 1.5 hours.

At the end of such addition, the whole was kept under stirring at 80°C during 2 hours.

Then, it was cooled down to room temperature.

100 g of the so-obtained product were dissolved, at 5°C, in 200 g of ethyl alcohol.

The mixture was percolated, during 60 minutes, into a solution of 28.0 g (0.27 mole) of diethylene triamine in 100 g of ethyl alcohol.

Stirring was carried out for half an hour at room temperature.

The ethyl alcohol was distilled until a temperature of 120°C and a residual pressure of 10 mm Hg were obtained.

There were obtained 128.0 g of a product having an amine number corresponding to 238 mg KOH/g, and having the formula:

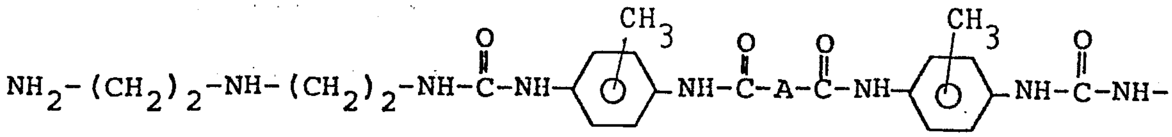

$-(CH_2)_2-NH-(CH_2)_2-NH_2$

wherein A is polyethylene glycol with a molecular weight of 400.

EXAMPLE 2

348.0 g (2 moles) of toluene diisocyanate (TDI) were introduced into a 4-neck flask having a capacity of 2,000 ml, equipped with stirrer, reflux cooler, dropping funnel and thermometer.

It was heated to 80°C and 1010.0 g (1 mole) of polypropylene glycol having a molecular weight of 1,000 were added in 1.5 hours.

On completion of such addition, the whole was kept at 80°C under stirring during 2 hours.

It was cooled down to room temperature.

100 g of the resulting product were dissolved, at 5°C, in 200 g of ethyl alcohol and then were gradually added, in 60 minutes, to a solution of 30.0 g of diethylene triamine in 100 g of ethyl alcohol.

The whole was left under stirring during half an hour at room temperature.

Ethyl alcohol was distilled until a temperature of 120°C and a residual pressure of 10 mm Hg were reached.

Obtained were 121.0 of a product having an amine number corresponding to 237 mg KOH/g, and having the formula:

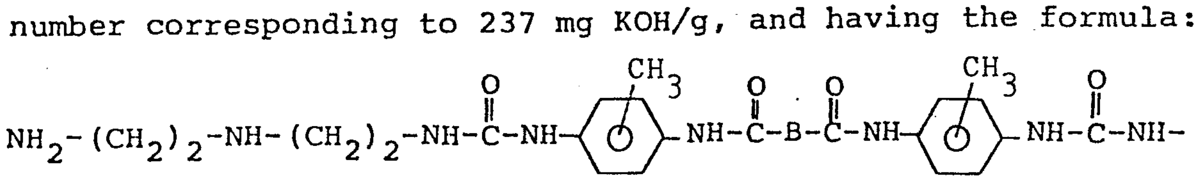

$-(CH_2)_2-NH-(CH_2)_2-NH_2$

in which B is polypropylene glycol having a molecular weight of 1,000.

## EXAMPLE 3

150.0 g of the intermediate product of Example 2 obtained from the reaction of toluene diisocyanate with polypropylene glycol having a molecular weight of 1,000, were dissolved in 350 g of ethyl alcohol. The solution was added to a solution of 23 g of diethylene triamine dissolved in 100 g of ethyl alcohol.

Ethyl alcohol was distilled until a temperature of 120°C and a residual pressure of 10 mm Hg were reached.

There were obtained 173 g of a product having an amine number corresponding to 165 mg KOH/g and a formula like the one of Example 2.

## EXAMPLE 4

A formulation was prepared by mixing, at room temperature, diglycidyl ether of bisphenol A, having an equivalent weight per epoxy oxygen equal to about 200, with a polyamidoamine of formula (1) of the type and in the amounts as shown in Table I.

The formulation was spread onto aluminum test pieces having a 3 mm thickness and a 25 mm width, which were coupled with each other with a 12 mm overlapping and by means of a pressure of about 15 $N/cm^2$, at 23°C. Under these conditions, hardening took place in 48 hours.

The characteristics of the formulation and of the joints after hardening are reported in Table I.

EXAMPLE 5

A formulation was prepared by mixing, at room temperature, the triglycidyl derived from para-amino-phenol having an equivalent weight per epoxy oxygen equal to about 103, with a polyamidoamine of formula (1) of the type and in the amounts indicated in Table II.

The operative modalities for the preparation of the test pieces are like those of Example 4 and the characteristics of the formulae are reported in Table II.

TABLE I

| Polyamidoamine of Example No. | 1 | 1 | 1 | 2 | 2 | 2 |
|---|---|---|---|---|---|---|
| Weight ratios: Epoxy resin: poly-amidoamine | 1:1 | 1:1.25 | 1:1.6 | 1:0.8 | 1:1 | 1:1.2 |
| Pot life (*) at 23°C, in minutes | 60 | 50 | 45 | 50 | 40 | 35 |
| Resistance to shearing tensile stress (**) in daN/$cm^2$ at temperatures of: | | | | | | |
| +237°C | 115 | 127 | 162 | 131 | 127 | 121 |
| + 80°C | 54 | 23 | 6 | 40 | 45 | 27 |
| -30 °C | 107 | 113 | 111 | 139 | 142 | 152 |

(*) Determined according to standard ASTM D 1338-56, re-approved in 1977 - Procedure A

(**) Determined according to standard ASTM D 1002-72 re-approved in 1978

TABLE II

| | | | | |
|---|---|---|---|---|
| Polyamidoamine of Example No. | 1 | 1 | 2 | 2 |
| Weight ratio: Epoxy resin: polyamidoamine | 1:1.5 | 1:2.3 | 1:2.5 | 1:4 |
| Pot life(*) at 23°C in minutes | 60 | 50 | 75 | 60 |
| Resistance to shearing tensile stress(**) in $daN/cm^2$ at a temperature of 23°C | 80 | 89 | 11 | 97 |
| Shore hardness A of the hardened formulate determined at 23°C | 97-99 | 97-99 | 94-97 | 90-92 |

(*) as in Table I
(**) as in Table I

CLAIMS:

1. A polyamidoamine having the formula:

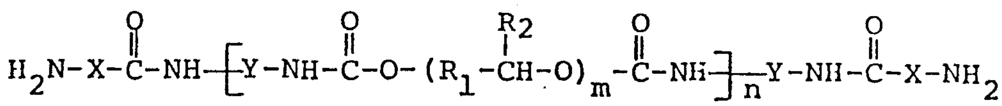 (1)

in which: $R_1$ is an alkylene radical containing from 1 to 6 carbon atoms; $R_2$ is hydrogen or an alkyl radical containing from 1 to 3 carbon atoms; X is the radical $-[(CH_2)_a-NH]_b$ or the radical

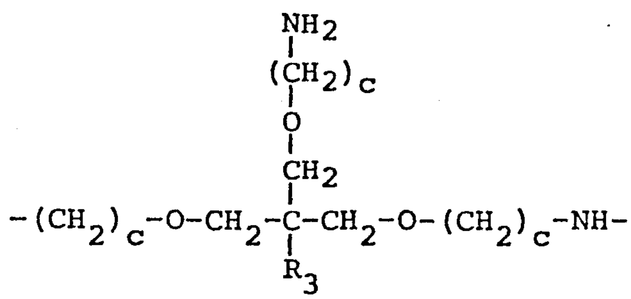

Y is a simple or substituted, linear or ramified aliphatic radical containing from 1 to 10 carbon atoms; a simple or substituted aryl radical containing from 6 to 20 carbon atoms; a simple or substituted cycloaliphatic radical containing from 3 to 20 carbon atoms, or an aliphatic-aromatic radical; m is an integer ranging from 1 to 100; n is an integer ranging from 0 to 10; a is an integer ranging from 2 to 6; b is an integer ranging from 1 to 6; c is an integer ranging from 1 to 4 and $R_3$ is an alkyl radical containing from 1 to 18 carbon atoms.

2. A polyamidoamine according to claim 1, in which Y is an alkylene-aromatic radical.

3. A process for preparing the polyamidoamine of formula (1) according to one or both of the claims 1-2, consisting in first preparing the -NCO terminated polyurethane resin according to scheme:

$$(n + I)\ OCN-Y-NCO\ (II) + nHO-\left[R_1-\overset{R_2}{\overset{|}{CH}}-O\right]_m H\ (III) \longrightarrow$$

$$\longrightarrow OCN-\left[Y-NH-\overset{O}{\overset{\|}{C}}-O-(R_1-\overset{R_2}{\overset{|}{CH}}-O)_m-\overset{O}{\overset{\|}{C}}-NH\right]_n Y-NCO\quad (IV)$$

and in subsequently reacting the resulting polyurethane resin with a polyamine according to the scheme:

$$OCN-\left[Y-NH-\overset{O}{\overset{\|}{C}}-O(R_1-\overset{R_2}{\overset{|}{CH}}-O)_m-\overset{O}{\overset{\|}{C}}-NH\right]_n Y-NCO\quad (IV) + 2\ H_2N-X-H\ (V) \longrightarrow$$

$$\longrightarrow H_2N-X-\overset{O}{\overset{\|}{C}}-NH-\left[Y-NH-\overset{O}{\overset{\|}{C}}-O(R_1-\overset{R_2}{\overset{|}{CH}}-O)_m-\overset{O}{\overset{\|}{C}}-NH\right]_n Y-NH-\overset{O}{\overset{\|}{C}}-X-NH_2\ .$$

4. The process according to claim 3, in which the polyurethane -NCO terminated resin (IV) is prepared by percolating polyglycol (III) into isocyanate (II) at a temperature ranging from 30 to 100°C.

5. The process according to one or both of the claims 3-4, in which the polyamidoamine of formula (1) is obtained by percolating a solution of polyurethane resin (IV) into a solution of polyamine (V) at a temperature ranging from 20 to 40°C.

6. A formulation comprising 100 parts by weight of an epoxy resin and from 50 to 250 parts by weight of a polyamidoamine of formula (1) as claimed in one or both of the claims 1-2.

7. A formulation according to claim 6 and comprising from 80 to 180 parts by weight of the polyamidoamine.